Europäisches Patentamt

European Patent Office

Office européen des brevets

(11) Publication number: **0 416 967 B1**

# EUROPEAN PATENT SPECIFICATION

(45) Date of publication of patent specification: **28.12.94**  (51) Int. Cl.5: **C12N 15/57**, C12N 9/56, C11D 3/386

(21) Application number: **90402220.9**

(22) Date of filing: **02.08.90**

(54) **Novel alkaline protease.**

(30) Priority: **11.08.89 JP 209380/89**

(43) Date of publication of application:
**13.03.91 Bulletin 91/11**

(45) Publication of the grant of the patent:
**28.12.94 Bulletin 94/52**

(84) Designated Contracting States:
**DE FR GB**

(56) References cited:
**EP-A- 0 251 446**

**THE JOURNAL OF BIOLOGICAL CHEMISTRY, vol. 260, no. 11, June 1985,pages 6518-6521, Baltimore, US; D.A. ESTELL et al.: "Engineering an Enzyme by Site-directed Mutagenesis to Be Resistant to Chemical Oxidation"**

**THE JOURNAL OF BIOLOGICAL CHEMISTRY, vol. 244, no. 19, October 1969, pages 5333-5338, Baltimore, US; C.E. STAUFFER et al.: "The Effect on Subtisilin Activity of Oxidizing a Methionine Residue"**

(73) Proprietor: **MITSUBISHI GAS CHEMICAL COM-PANY, INC.**
**No. 5-2, Marunouchi 2-chome**
**Chiyoda-ku,**
**Tokyo (JP)**

(72) Inventor: **Morino, Kazuhiko**
**182, Shinwari,**
**Tayuhama**
**Niigata-shi,**
**Niigata 950-31 (JP)**
Inventor: **Otsuka, Yoshimi**
**182, Shinwari,**
**Tayuhama**
**Niigata-shi,**
**Niigata 950-31 (JP)**
Inventor: **Tahara, Torakazu**
**182, Shinwari,**
**Tayuhama**
**Niigata-shi,**
**Niigata 950-31 (JP)**

(74) Representative: **Bourgognon, Jean-Marie et al**
**Cabinet Flechner**
**22, Avenue de Friedland**
**F-75008 Paris (FR)**

EP 0 416 967 B1

NUCLEIC ACIDS RESEARCH, vol. 13, no. 24, 1985, pages 8913-8926, Oxford, GB; M. JACOBS et al.: "Cloning, sequencing and expression of subtisilin Carlsberg from Bacillus licheniformis"

TRENDS IN BIOCHEMICAL SCIENCES, vol. 13, August 1988, pages 291-297, Cambridge, GB; J.A. WELLS et al.: "Subtisilin - an enzyme designed to be engineered"

**Description**

Background of the invention

The present invention relates to a novel alkaline protease, particularly a novel alkaline protease wherein one or two specific amino acids in an alkaline protease originated from Bacillus licheniformis are substituted by the other one or ones thereby resulting in having superior properties. The present novel alkaline protease is useful for an auxiliary cleaning agent which is admixed in detergents or a protein-removing agent of contact lenses.

Proteases useful for auxiliary cleaning agents in detergents or protein-removing agents of contact lenses are selected from those which exist in nature. Proteases originated from some bacteria belonging to, for example, Bacillus are used for auxiliary cleaning agents in detergents and papain or alkaline proteases originated from some bacteria belonging to, for example, Bacillus for protein-removing agents of contact lenses.

Proteases useful for auxiliary cleaning agents in detergents have to satisfy such many requirements as high activity under pH 10 - 11, stability against ingredients of detergents such as surface active agents and/or builders, pH stability within the wide range, stability against bleaching agents, wide substrate specificities and the like. Proteases available in these days are readily degraded by surface active agents and/or bleaching agents which are principal ingredients in detergents. One of approaches for stabilizations of proteases is granulation with, for example, coating. This approach is hardly applied to liquid detergents, so that stabilization of liquid detergents containing proteases is difficult. Although alkaline proteases resistant against such oxidation agents as peroxide bleaching agents are useful, the natural alkaline proteases have so little resistance against such oxidation agents that application thereof is restricted. Little resistance of the natural alkaline proteases against oxidation agents is due to the fact that methionine at the 222nd position is oxidized by the oxidation agents until it is converted to methioninesulfoxide (C.E. Stauffer, Don Etson: J. Biol. Chem., 244, 5333, 1969). Mutated enzymes hardly influenced by oxidation agents would be expected by substituting other amino acids which are inert against oxidation agents for the methionine at the 222nd position. On the basis of those facts, it is reported that mutated enzymes where the methionine at the 222nd position of alkaline protease originated from Bacillus amyloliquefaciens is substituted by other 19 amino acids are created. (D.A. Estell et al: J. Biol. Chem. 260, 6518, 1985; US 4760025).

Substitution of the other amino acids for amino acids in natural enzymes often induces decrease in specific activity due to change in three dimensional structures of the enzymes. One of the mutated enzymes mentioned above, e.g., alanine-substituted enzyme which has the greatest specific activity among mutants, is not satisfactory, since specific activity thereof is 53% as high as the wild enzyme. Decrease in specific activity makes it difficult to use the mutated enzymes in place of the wild enzymes, even though resistance against oxidation agents is improved by substitution of amino acids. Practical application field of mutated enzymes is rather narrowed than wild enzymes. Enzymes which have strong resistance against oxidation agents as well as specific activity similar to the wild enzymes, at the least, are demanded from a practical point of view.

Alkaline proteases commercially used for detergents are those originated from Bacillus, e.g., Bacillus licheniformis, Bacillus amyloliquefaciens or Bacillus subtilis. Those alkaline proteases are superior to the others for detergents in many respects, although they have difficulties mentioned above. After study was made on the basis of the alkaline proteases originated from Bacillus in order to obtain mutated enzymes useful for detergents, the present inventors have found that substitution of aspartic acid for glycine at the 105th position of alkaline protease originated from Bacillus licheniformis improves resistance against surface active agents. We have also found that substitution of glutamine for methionine at the 221st position of alkaline protease originated from Bacillus licheniformis wherein an amino acid at the 222nd position is not methionine but alanine makes enzyme have almost the same specific activity as that of the wild type enzyme and be stable against oxidation agents. In addition, we have found that only substitution of glutamine for the methionine at the 221st position increase stability against oxidation agents but degrades stability against surface active agents, but substitution of aspartic acid for the glycine at the 105th position as well as substitution of glutamine for the methionine at the 221st position recover stability against surface active agents.

According to the present invention, a novel alkaline protease is provided, i.e., an alkaline protease originated from Bacillus licheniformis wherein the glycine at the 105th position is substituted by aspartic acid and/or the methionine at the 221st position is substituted by glutamine. The 105th or 221st position means a position counting from the N-terminal amino acid of mature protein of the alkaline protease. The mutated enzyme having improved resistance against surface active agents is obtained by cloning an alkaline protease gene from a microorganism belonging to Bacillus mentioned above, treating the cloned gene with a mutation-inducing agent, preparing a mutated enzyme gene which codes an enzyme having different amino acid or acids from those of the natural enzyme at some position or positions, culturing a transformant having the mutated enzyme gene and screening an alkaline protease having improved resistance against surface active agents from proteases produced. Difference in structures between the natural enzyme and the modified one is cleared up by sequence determination for nucleic acids. A mutated enzyme having resistance against oxidation agents as well as high specific activity is obtained by applying a site-directed mutagenesis to the alkaline protease gene until the methionine therein which is to be oxidized by oxidation agents is substituted by the other amino acid, and screening the desired one.

The above procedure is explained hereinunder in more detail.

The alkaline protease gene of Bacillus licheniformis is isolated according to JP 63-214187. That is, the isolation is made by shot gun cloning where a host is a low protease-producing strain belonging to Bacillus subtilis, or colony hybridization wherein a probe is DNA homologous with the alkaline protease gene. The shot-gun cloning for obtaining a strain having the alkaline protease gene is conducted in such a manner that fragments obtained by digestion of chromosome DNAs of Bacillus licheniformis with restriction enzymes are ligated to a vector DNA of microorganisms belonging to Bacillus, for example, such plasmids as pUB 110 or pE 194, or a shuttle vector of both microorganisms belonging to Bacillus and Escherichia coli, such as pHY 300 PLK until transformation of a low protease-producing strain of Bacillus subtilis is effected and then transformants in which capability of producing proteases are improved are selected.

Transformation of Bacillus subtilis by recombinant DNAs is conducted by a protoplast method (S. Chang, S. N. Cohen: Molec. Gen. Genet., 168, 111, 1979) or a method using competent cells (D. Dubnau, R. D. Abelson: J. Mol. Biol., 56, 209, 1971). The former is conducted in such a manner that a lysozyme treatment is applied until protoplasts are produced and the DNAs are introduced in the presence of polyethyleneglycol. The latter is conducted in such a manner that the microorganism is cultured in a minimum medium containing glucose in order to make a modification so that the DNAs are easily introduced.

The colony hybridization is conducted using the DNAs mentioned below as a probe. Single stranded oligonucleotides may be employed which have usually 14 to 18 nucleotides wherein nucleotide secuence corresponds to sequence of about 5 to 6 amino acids connected in series at any portion of a known amino acid sequence in alkaline proteases originated from Bacillus licheniformis (M. Ottensen. I. Svendsen: Methods in Enzymology, 19, 199, Academic Press, New York, 1979), for example, a mixture of 16 kinds of oligonucleotides having 14 nucleotides each having nucleotide sequence as follows:

ATGAA(X)CA(Y)GC(Z)GT

wherein X and Y: same or different; A or G

Z : T, C, A or G

which corresponds to the sequence of amino acids

Met-Lys-Gln-Ala-Val

at from the 134th to the 138th positions from the N-terminal amino acid. When a homologous gene to the desired alkaline protease is obtained, this gene may be employed as a probe. After ligating chromosome DNA fragments to Escherichia coli plasmid, for example, pBR 322 in accordance with the same procedure as in the shot gun cloning in order to transform competent cells of E. coli which have been modified to be ready for introduction of DNAs by means of, for example, a treatment with $MnCl_2$-$CaCl_2$, and a strain carring alkaline protease gene is isolated by colony hybridization with a probe, i.e., the homologous DNAs labeled with [32]p (M. Grunstein, J. Walls: Methods in Enzymology, 68, 379, Academic Press, New York, 1979).

A recombinant which is able to produce the alkaline protease is obtained, by recloning the obtained alkaline protease gene into plasmids of Bacillus subtilis and transforming Bacillus subtilis.

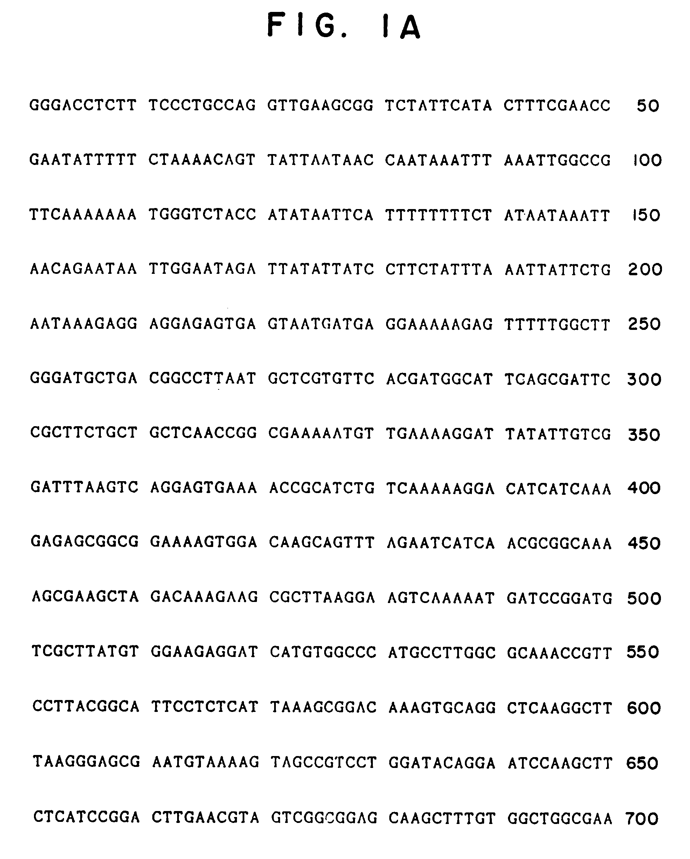

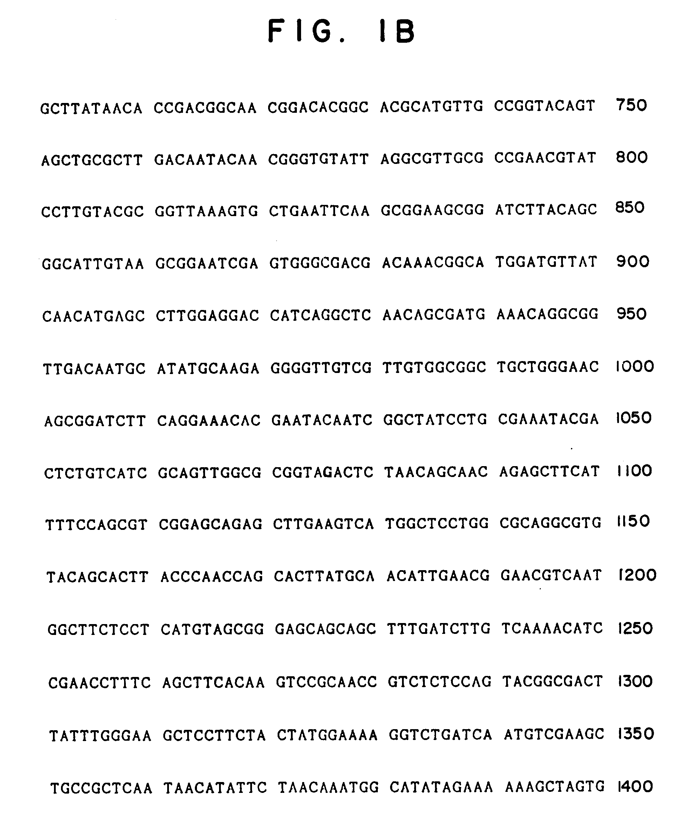

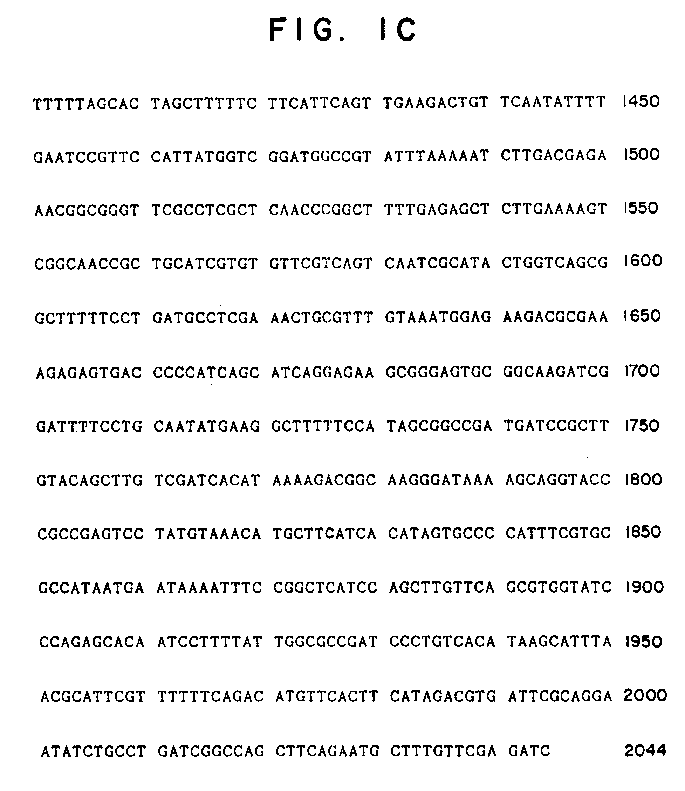

In the accompany drawings, Fig. 1 is whole nucleotide sequence of alkaline protease gene originated from Bacillus licheniformis HP 19611,

Figs. 2, 3, 4 and 5 are amino acid sequences in a wild type alkaline protease, an alkaline protease resistant against surface active agents, an alkaline protease resistant against oxidation agents and an alkaline protease resistant against both surface active agents and oxidation agents, respectively,

Fig. 6 is a restriction map of a recombinant plasmid pHLP 352,

Fig. 7 is a restriction map of a recombinant phage MI3LPI,

Fig. 8 is a restriction map of a recombinant plasmid pULP 355, and

Fig. 9 is nucleotide sequence of a primer for substituting glutamine for the methionine at the 221st position.

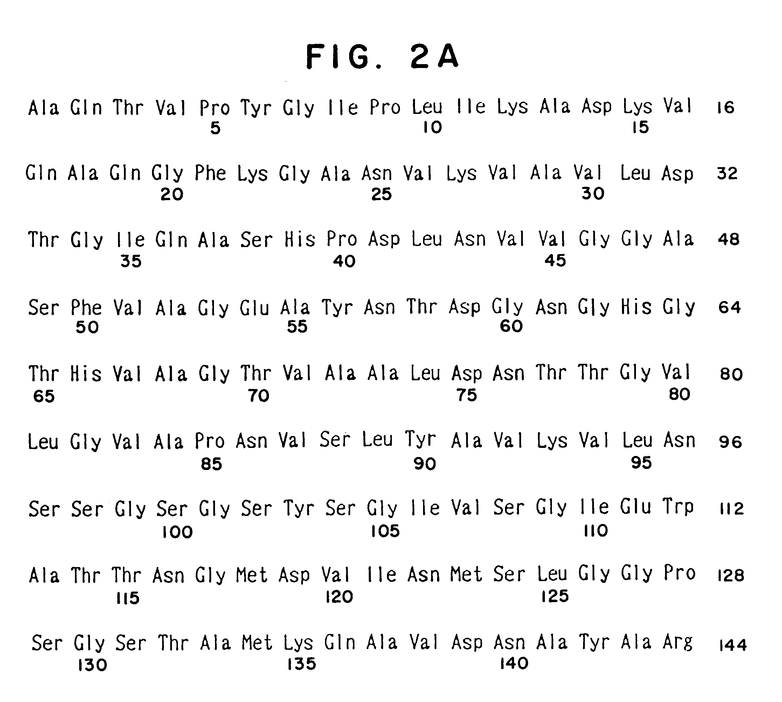

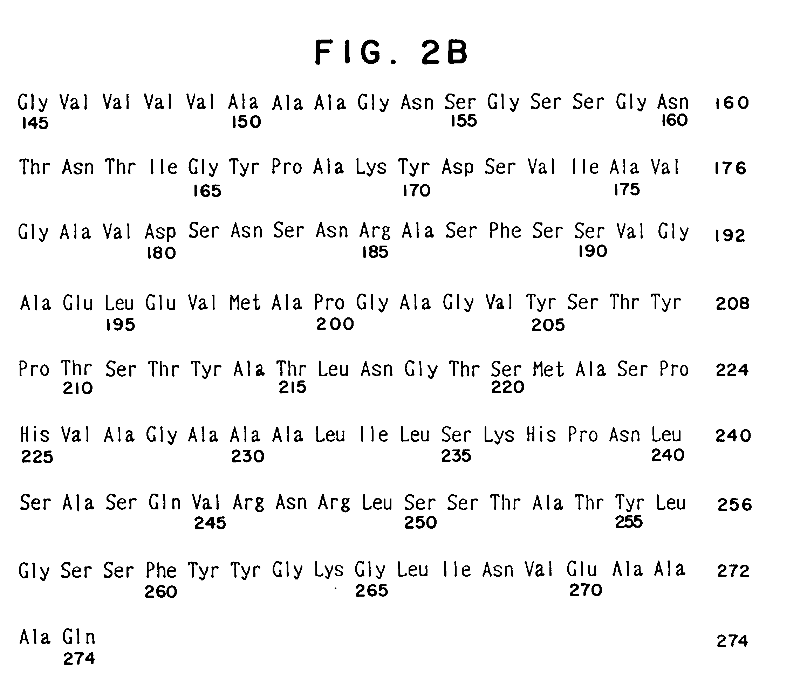

In Fig. 2 - 5, and 9,

Gly = glycine

Val = valine,

Ile = isoleucine,

Thr = threonine,

Glu = glutamic acid,

Gln = glutamine,

Arg = arginine,

Phe = phenylalanine,

Trp = tryptophan,

Ala = alanine,

Leu = leucine,

Ser = serine,

Asp = aspartic acid,

Asn = asparagine,

Lys = lysine,

Met = methionine,

Tyr = tyrosine,

His = histidine and

Pro = proline

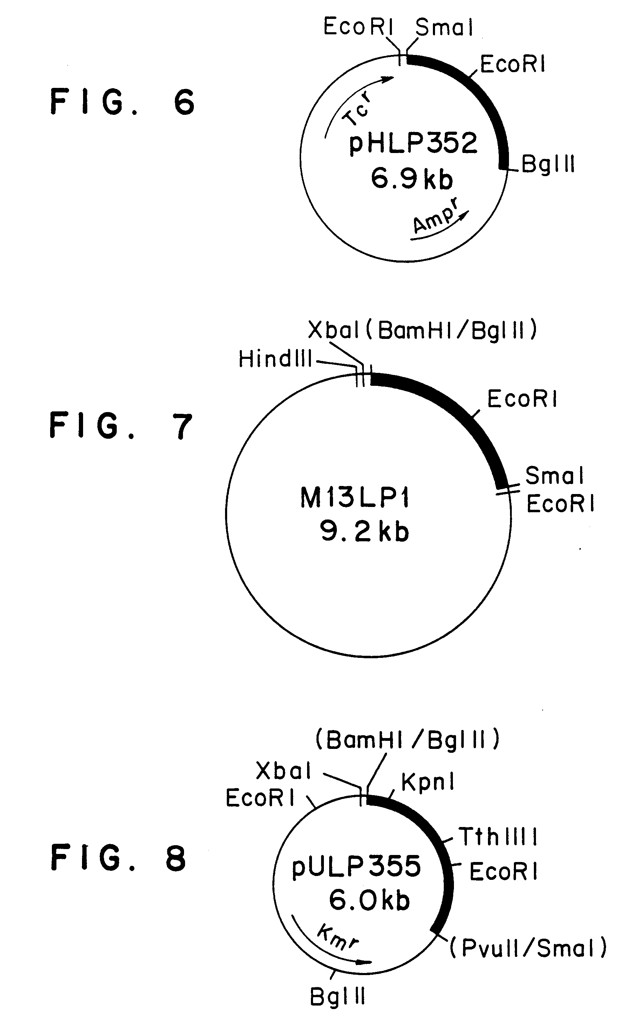

In Fig. 6 - 8, thin lines are areas for vector plasmids, thick lines are areas originated from chromosome DNAs and arrows show areas on vector plasmids where antibiotics-resistant genes are present.

Nucleotide sequence in the alkaline protease gene obtained by the procedures mentioned above is shown in Fig. 1 wherein a code of mature protein of alkaline proteinase lies in the area from 539th to 1360th. Amino acid sequence in mature protein of the alkaline protease derived from the nucleotide sequence of Fig. 1 is shown in Fig. 2. The gene above is originated from Bacillus licheniformis strain, HPl9611 (FERM BP 3020).

A mutated alkaline protease is obtained by, for example, causing mutation in the alkaline protease. A DNA fragment which encodes alkaline protease are ligated with, replicative form DNAs (RF-DNA) of single stranded phage, for example such as Ml3mpl8 of Ml3mpl9 in order to transform Escherichia coli, e.g., E. coli JM109, and the transformant is overlayed on an H-agar plate* containing X-gal (5-bromo-4-chloro-3-indolyl-$\beta$-D-galactoside).

```
* polypeptone          1% by weight,

  sodium chloride      0.8% by weight,

  agar                 1.2% by weight
```

Phages producing colorless plaque are collected and E. coli is allowed to be infected therewith. RF-DNA is extracted from the infected E. coli and phages having DNAs of a given length are selected. E. coli is infected with the phages selected in the same manner as above and a single stranded DNA is obtained from phages present in the supernatant of the culture. The single stranded phage DNA is treated with mutation-inducing agents, e.g., sodium nitrite, sodium sulfite or hydroxylamine, and then universal primer having complementary sequence to the M 13 phage DNA is added thereto. The mixture is left to stand at 60 - 70°C for 20 - 60 min. and then at room temperature for 20 - 60 min., until the primer is annealed to the phage DNA. The treatment with mutation-inducing agents mentioned above is preferably conducted under the conditions that substitution of at least one base in the alkaline protease gene is induced. In case of, for example, sodium nitrite, the treatment is conducted at room temperature for 2 - 5 min. for such a concentrated agent as 1 M, and at a room temperature for 20 - 40 min. for such a dilute agent as 75 mM.

Four varieties of nucleotides, i.e., dATP, dGTP, dCTP and dTTP (the final concentration: 0.1 - 0.3 mM each) and a Klenow fragment of 1 - 5 units/$\mu$g DNA are added to the phage and the mixture is left to stand at 30 - 37°C for 60 - 120 min. until DNA is elongated.

The DNA is digested with restriction enzymes to obtain a fragment having the whole of the alkaline protease gene and the fragment is ligated to plasmids of Bacillus subtilis until Batillus subtilis is transformed. Alternatively, a fragment having a part or the whole of the mature protein area of the alkaline protease is cleaved and then is ligated to the corresponding area in a recombinant plasmid to which the alkaline protease gene has been introduced, until Bacillus subtilis is transformed. The transformant is inoculated on a nutrient broth agar plate * containing 1% or so by weight of casein and cultured overnight at 37°C in order to select transformed cells which are able to produce halos.

```
* Peptone                   1% by weight,
  Meat extract              0.5% by weight,
  Sodium chloride           0.5% by weight,
  Agar                      1.2% by weight,
  pH                        7.4.
```

Selection of a strain capable of producing surface active agents-resistant proteases is made in such a manner that the halo-producing strains are cultured, protease activities of supernatant are measured in the presence or absence of surface active agents and strains wherein ratios of activities in the presence of the surface active agents to those in the absence thereof are greater than those of wild strains are selected. Mutation sites of mutated alkaline proteases are identified on the basis of nucleotide sequence by, for example, a dideoxy sequencing method which is familiar to the skilled in the art.

Isolation of a mutant gene which codes alkaline protease having glutamine substituted for the methionine at the 221st position is effected, for example, in such a manner that synthetic oligonucleotide composed of 20 - 40 nucleotides having sequence correspond to the sequence of amino acids at about three to six residues in both up stream and down stream from the 221st position amino acid except a codon for glutamine is substituted for that for methionine at the 221st position, and site-directed mutagenesis is carried out using the oligonucleotide as a primer in a commercially available kit, for instance, "mutagen" (trade name; Bio-Rad Co.) as mentioned below. That is, a fragment containing the alkaline protease gene is ligated with, replicative form DNA (RF-DNA) of single stranded DNA phage, for example, Ml3mp18 or M13mp19 in order to transform E. coli e.g., E. coli JM 109, the E. coli is overlayed on the H-agar plate containing X-gal, a phage which is able to produce colorless-plaques is collected, the E. coli is infected with the phage, RF-DNA is extracted from the infected E. coli, and a phage having a DNA of a given length is selected. E. coli CJ 236 is infected with the phage thus selected and a singled strand phage DNA containing uracil from phage particles in supernatant of a culture. To the single stranded phage DNA is annealed the synthetic oligonucleotide mentioned above whose 5′-end is phosphorylated. Then, four kinds of nucleotides i.e., dATP, dGTP, dCTP and dTTP having final concentration each of 0.2 - 1 mM and T4DNA polymerase of 1 - 5 units/$\mu$g DNA are added thereto. The mixture is left to stand at 30 - 37°C for 60 - 120 min., in order to effect elongation of DNA. E. coli MV 1190 is transformed with the reaction solution according to a calcium chloride method which is familiar to the skilled in the art, until plaques are obtained. Single stranded DNAs are prepared from any ten plaques and DNA whose nucleotide sequence at the mutated site determined by the dideoxy sequencing method is the same as that of synthetic oligonucleotide is selected.

Production of mutated alkaline protease by use of the mutated alkaline protease gene obtained by site-directed mutagenesis mentioned above is carried out in such a manner that, for example, a RF-DNA is prepared from E. coli infected with the phage mentioned above, the RF-DNA is digested with restriction enzymes to cleave a fragment having the whole of the alkaline protease gene and then the fragment is ligated to Bacillus subtilis plasmids until Bacillus subtilis is transformed. Alternatively, a fragment having a part or the whole of the mature protein area of the alkaline protease is substituted for the corresponding area in a recombinant plasmid to which the alkaline protease gene has been introduced in order to transform Bacillus subtilis and then the transformant is cultured in a usual manner.

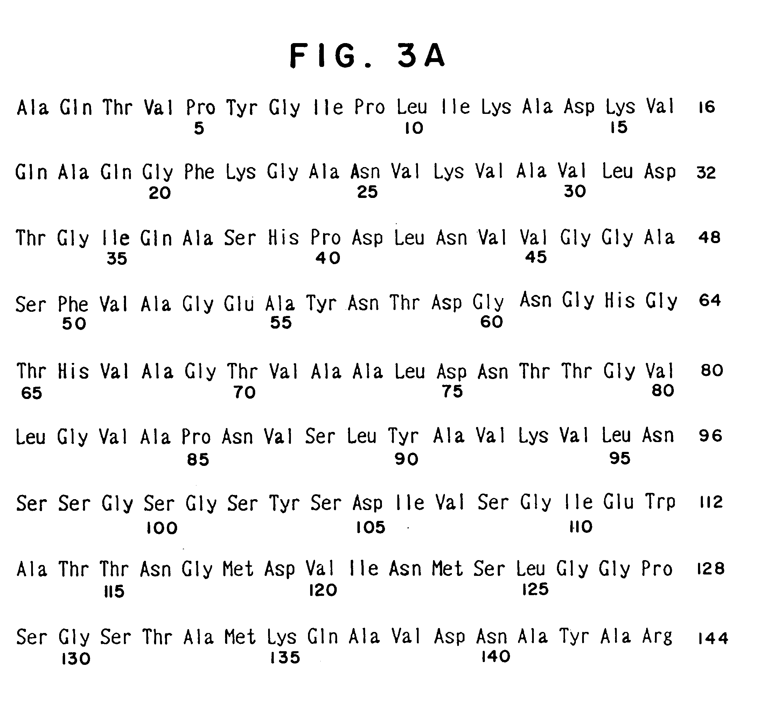

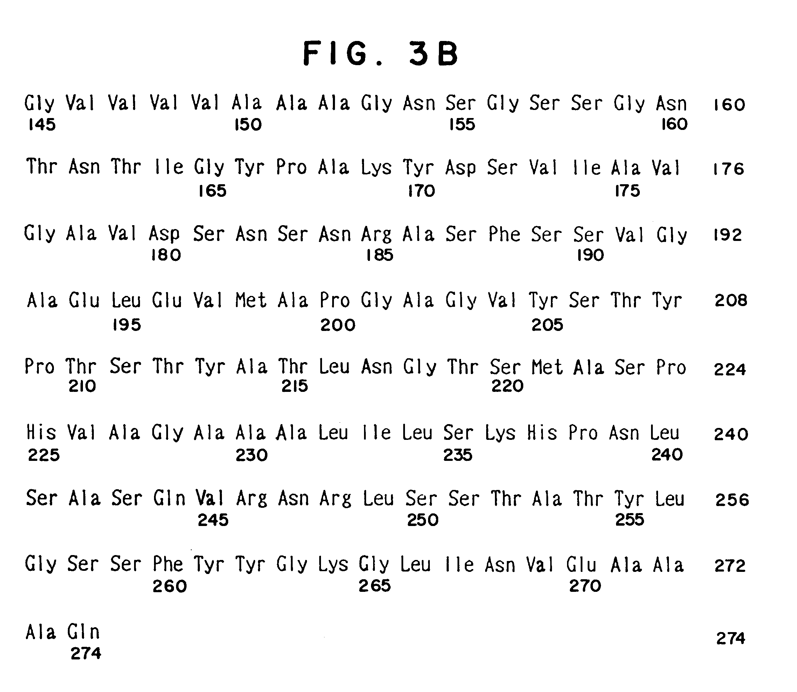

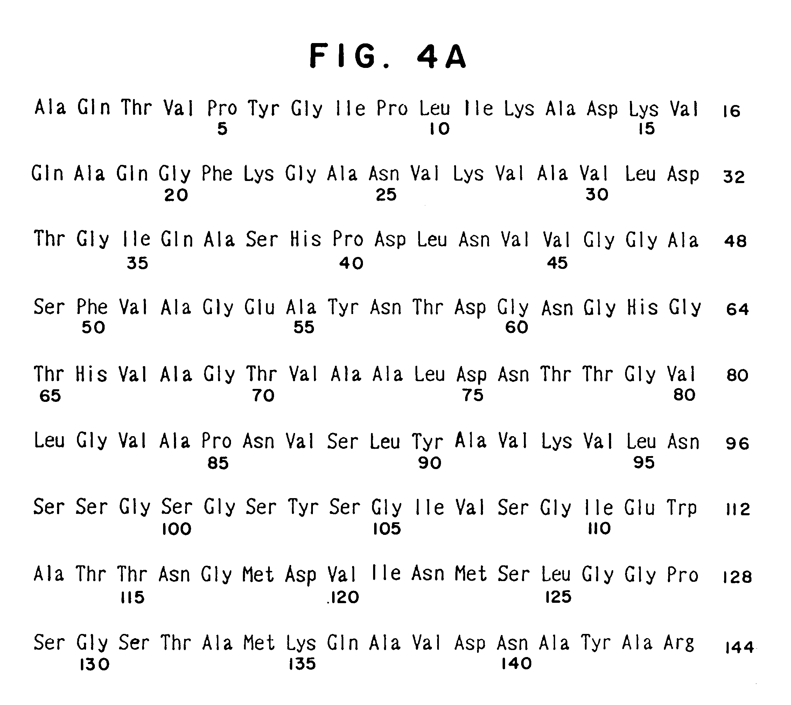

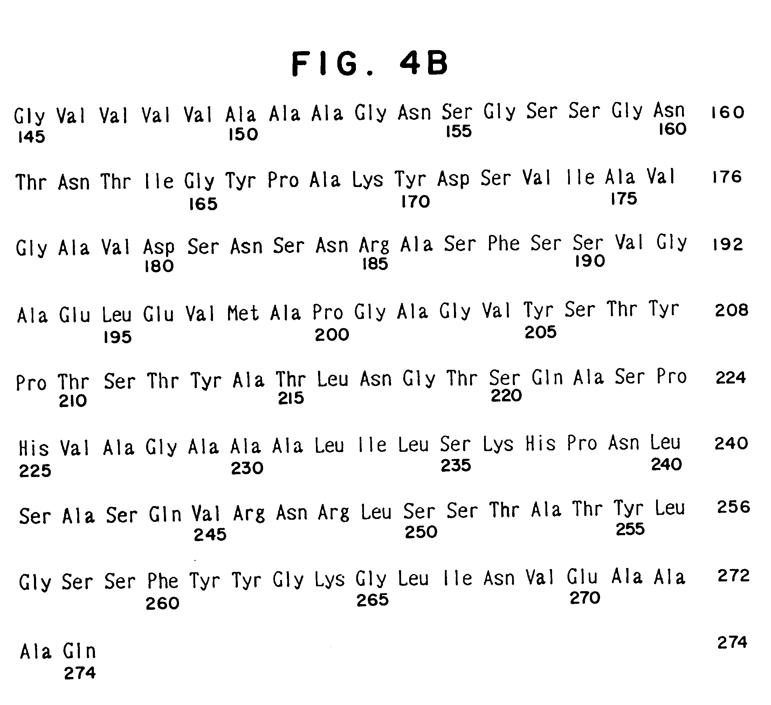

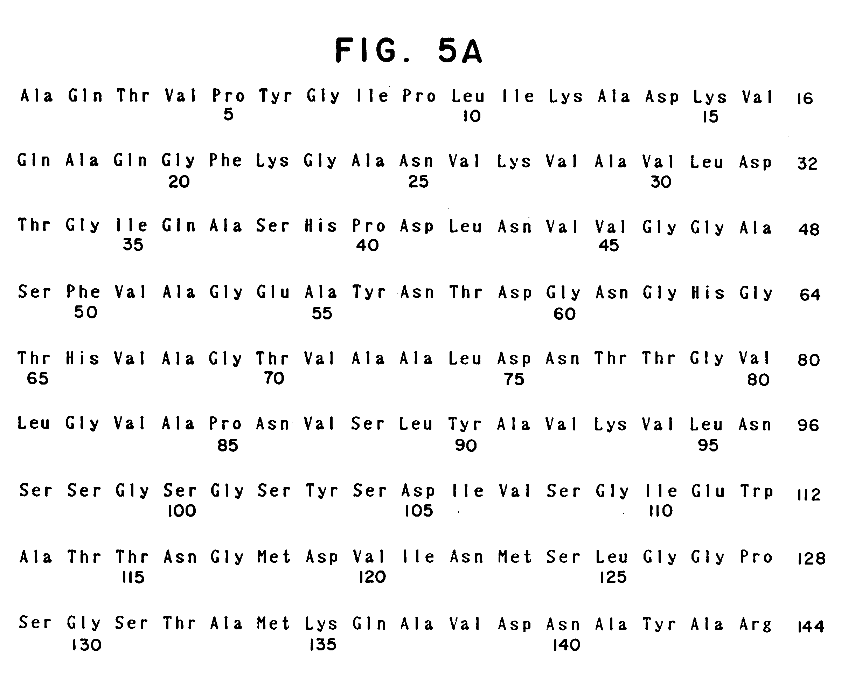

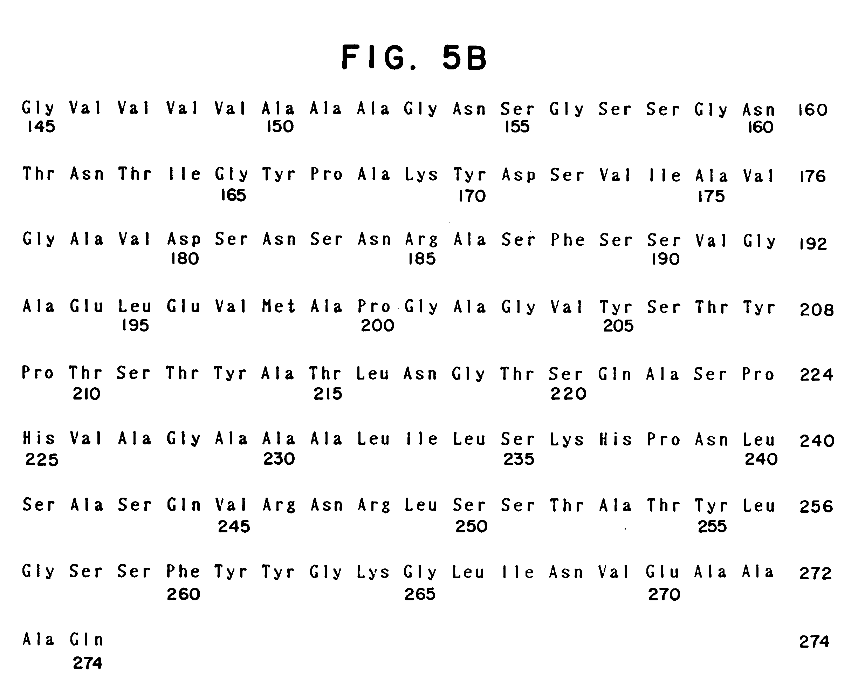

Amino acid sequence of the present alkaline protease resistant against surface active agents and that of the alkaline protease having high specific activity as well as resistance against oxidation agents are shown in Fig. 3 and Fig. 4, respectively. Fig. 5 is an amino acid sequence in an alkaline protease resistant against both surface active agent and oxidation agents. Difference from amino acid sequence of natural alkaline protease as shown in Fig. 2 lies only in amino acids located at the 105th and the 221st position, respectively.

One of the present alkaline proteases, i.e., that resistant against surface active agents, has activity of 45% as strong as that when no linear alkylbenzene sulfonate (hereinafter referred to as LAS) is present, even in the presence of 0.12% by weight of LAS. The resistance is greater than that of the natural alkaline protease, i.e., 36%. Specific activity of the mutated enzyme which is measured with casein as a substrate is 7860 unit/mg protein.

Another alkaline protease, i.e., the mutated enzyme resistant against oxidation agents and having high specific activity, has 7170 units/mg protein of specific activity which is almost the same level as 7680 units/mg protein of the natural alkaline protease. Residual activity after a treatment with 1 M hydrogen peroxide, i.e., resistance against hydrogen peroxide, of the present alkaline protease resistant against oxidation agents, is 94% while 14% of the natural alkaline protease.

The protease resistant against both oxidation agents and surface active agents is obtained by recombination of the respective mutated protease genes in order to prepare a gene having both mutated sites. Alternatively, a gene having both mutated sites is prepared by site-directed mutagenesis using either mutated gene as a template. Then, the transformant is cultured in the same manner as mentioned above.

The alkaline protease resistant against both surface active agents and oxidation agents has 6200 units/mg protein of specific activity, and 94% of resistance against hydrogen peroxide. Activity in the presence of LAS, for example, is 28% as much as that in the absence of LAS.

The following examples explain the present invention in more detail. Any enzymes commercially available such as enzymes produced by Takara Shuzo Co. Ltd., Japan or Toyobo Co. Ltd., Japan may be employed with respect to restriction enzymes, T4DNA ligases and Klenow fragments. Treatments of DNAs with these enzymes may be effected under the conditions mentioned in the catalogues of the enzymes. As to vector DNAs, any commercially available ones, may be employed. In addition, any products obtained by extraction or purification in a manner familiar to the skilled in the art from Bacillus subtilis or E. coli containing the DNA in issue may be used.

Example 1

Production of a surface active agent-resistant alkaline protease gene

A recombinant plasmid pHLP352 was prepared from Bacillus subutilis PW10 (pHLP352) (FERM BP 3021) in a usual manner. A recombinant plasmid pHLP352 (Fig. 6, 5 $\mu$g) where an alkaline protease gene (Fig. 1) of Bacillus licheniformis HP 19611 (FERM BP 3020 ) is joined to a vector pHY300PLK was digested with Sma I and Egl II (10 units, each). Fragments obtained were fractionated by agarose gel electrophoresis to obtain a 2.0 Kb fragment (about 1.4 $\mu$g). On the other hand, RF-DNA of Ml3mp19 (1 $\mu$g) was digested with Sma I and BamH I (2 units each), and heat-treated at 65°C for 10 min., and then DNAs were recovered by precipitation with ethanol. The both DNAs were mixed and ligated together with T4DNA ligase (2 units). Escherichia coli JM 109 was transformed with the ligated DNA (0.12 $\mu$g), and one recombinant phage which produces colorless plaque on the H-agar plate medium containing X-gal was obtained. The recombinant phage was named as MI3LPl, whose DNA restriction map is shown in Fig. 7.

Pre-cultured solution (50 $\mu$l) obtained by culturing Escherichia coli JM 109 overnight at 37°C in a 2 x TY medium (pH 7.4) containing 1.6% by weight ofbactotrypton, 1% by weight of yeast extract and 0.5% by weight of sodium chloride and plaque of the recombinant phage MI3LPl, mentioned above, was inoculated in the 2 x TY medium (5 ml). Culturing was made at 37°C for 5 hours in order to obtain a seed phage solution. To the 2 x TY medium (200 ml) were added the pre-cultured solution mentioned above (2 ml) and the seed phage solution (2 ml), and culturing was made at 37°C for 5 hours. Centrifugation gave supernatant (185 ml) containing phage particles.

To the supernatant was added 20% by weight of polyethyleneglycol 6000/2.5 M NaCl (37 ml) and the mixture was left to stand for 15 min. to precipitate phages. The precipitate obtained after centrifugation was suspended in 1 ml of TE buffer solution (10 mM tris-HCl buffer solution (pH 8.0)/0.1 mM EDTA). Phenol (1 ml, saturated with TE buffer solution) was added and thoroughly mixed until DNA was extracted. Centrifugation was made to recover an aqueous layer to which ethanol was added to obtain a single stranded phage DNA (about 200 $\mu$g).

To solution of the single stranded phage DNA (100 $\mu$g) in 0.5 M sodium acetate (pH 4.2, 500 $\mu$l) was added 2 M sodium nitrite (500 $\mu$l) and the mixture was left to stand at 20°C for 5 min. until mutation of the DNA was induced. DNA recovered by adding ethanol thereto was dissolved in the TE buffer solution (1 ml).

To the mutated DNA solution was added universal primer (72 p mol, 15 bases of oligonucleotide having complementary sequence to the M 13 phage DNA) and heating was made at 60°C for 20 min. before being left to stand at room temperature for 20 min. in order to effect annealing. Thereto were added four kinds of nucleotides, i.e., dGTP, dATP, dTTP and dCTP (182 p mol, each) and Klenow fragment (126 units) and elongation reaction was carried out at 30°C for 90 min. Heating was effected at 65°C for 10 min. in order to stop the reaction. After extraction with phenol was made, ethanol was added in order to collect a DNA.

The DNA was digested with Sma I and Xba I (500 units each) and subjected to fractionation by means of agarose gel electrophoresis in order to prepare a 2 Kb fragment. On the other hand, pUB 110 (125 $\mu$g) was digested with Pvu II and Xba I (250 units each) and was subjected to fractionation in the same manner as above until a 4Kb fragment was obtained. The both fragments were mixed and DNAs were ligated together by use of T4 DNA ligase (50 units). As a control, a 2 Kb fragment of unmodified MI3LPI digested with Sma I - Xba I and a 4 Kb fragment of pUB 110 digested with Pvu II - Xba I were ligated together in the same manner as above in order to prepare a plasmid pULP 355 containing wild type gene (Fig. 8).

Bacillus subtilis PWI0 (FERM BP 3019), a low protease-producing strain, was transformed with the ligated DNAs above as follows.

Bacillus substilis PW10 was inoculated in a nutrient broth (5 ml, pH 7.4) containing 1% by weight of peptone, 0.5% by weight of meat extract and 0.5% by weight of sodium chloride and cultured overnight. The cultured solution obtained (0.3 ml) was inoculated in the same nutrient broth (30 ml) as above and further cultured at 37°C for 1.5 hours. Cells were harvested by centrifugation and suspended in SMMP (2.5 ml), a 1 : 1 mixture of 2 x SMM* and 4 times concentrated Penassay broth (produced by Difco Co.)

| * | Succrose | 1 M |
|---|---|---|
| | Maleic acid | 0.04 M |
| | $MgCl_2$ | 0.04 M |
| | pH | 6.5 |

To the suspension was added lysozyme (5 mg) and the mixture was left to stand at 37°C for 2 hours until suspension of protoplast was obtained.

To the suspension (1.5 ml) were added the solution of the joined DNA mentioned above (2 $\mu$g) and 40% polyethyleneglycol 4000 solution (4.5 ml) in 2 x SMM, and the mixture was kept for 2 min. under gentle stirring. Thereto was added SMMP (15 ml). The protoplast was suspended in SMMP (3 ml) and the suspension was left to stand at 30°C for 1.5 hours.

The suspension of the protoplast (100 $\mu$l each) was spread on DM3 regeneration medium* containing Kanamycin (200 $\mu$g/ml) and the medium was left to stand at 37°C for two days to form colonies.

| * | sodium succinate | 0.5 M |
|---|---|---|
| | glucose | 0.5% by weight |
| | Casamino acid | 0.5% by weight |
| | yeast extract | 0.01% by weight |
| | bovine serum albumin | 0.01% by weight |
| | dipotassium hydrogen phosphate | 0.35% by weight |
| | potassium dihydrogen phosphate | 0.15% by weight |
| | $MgCl_2$ | 20 mM |
| | agar | 0.8% by weight |
| | pH | 7.3 |

The transformation mentioned above was repeated thirteen times until 14099 of Kanamycin-resistant transformants, were obtained. The transformants were subjected to a replica-plating method on a casein plate (a nutrient broth agar plate containing 1% by weight of casein), further containing Kanamycin (20 $\mu$g/ml) and culturing was made at 37°C for 16 hours to obtain halo-producing strains (4644 of strains). The halo-producing strains were subjected to a replica plating method in two nutrient broth agar plates and culturing was made at 37°C for 16 hours before colonies were peeled off. Nutrient broth agar containing 1% by weight of casein was laid on one of the two agar plates above and nutrient broth agar containing 0.12% by weight of LAS as well as 1% by weight of casein was laid on the other one of the two agar plates. Culturing was made at 37°C for one hour. Formation of halos in the two agar plates was compared with that of the strain (H4) having wild gene in order to select 261 strains which formed large halos from the medium containing LAS.

The strains selected were cultured in nutrient broth for 40 hours. Activities of supernatants of culture, after being diluted to suitable concentration, obtained in the presence or absence of LAS were measured as in the manner given below.

To a substrate solution (400 $\mu$l, 50 mM glycine/sodium hydroxide buffer solution (pH 10) containing 0.75 mM of N-succinyl-L-Ala-L-Ala-L-Pro-L-Phe-p-nitroanilide/1 mM of $CaCl_2$) and another substrate solution (400 $\mu$l) having the same composition as above but to which 0.18% by weight of LAS was added, respectively, were added the diluted solutions (200 $\mu$l each) mentioned above, respectively, and the mixtures were allowed to stand at 37°C for 20 min. before a reaction ceasing agent (100 $\mu$l, 0.35 M trichloroacetic acid/1.05 M acetic acid/0.7 M sodium acetate) was added. Absorbance at 410 nm of the reaction solutions after the reactions were ceased was measured. LAS-resistance degrees, i.e., ratio of absorbance of the reaction solution obtained in the presence of LAS to that of the reaction solution obtained in the absence of LAS was calculated with respect to all of the 261 strains. LAS resistance degree of wild type gene-containing strain (H4) was 36%, while that of one of mutant strains (75-Ll) was 45%.

Restriction map of plasmid pDTG75 of the strain, 75-Ll was the same as that of pULP355.

Example 2

Identification of mutated site of a surface active agent-resistant alkaline protease gene

Plasmid extracted from the strain 75-Ll obtained in example 1 was digested with Xba I and Bgl II and subjected to agarose gel electrophoresis for fractionation to prepare a 3.3 Kb fragment. On the other hand, pUCl9 was digested with Xba I and BamH I. The fragment was ligated with the 3.3 Kb fragment above. Escherichia coli LE 392 was transformed with the ligated DNA to obtain a strain resistant against ampicillin and a plasmid was prepared therefrom. Nucleotide sequence in the plasmid by means of a dideoxy sequencing method showed substitution of bases, i.e., substitution of A for G, which corresponds to substitution of aspartic acid (codon GAC) for glycine (codon GGC) at the 105th position of the alkaline protease.

Example 3

Site-directed mutagenesis of an alkaline protease gene

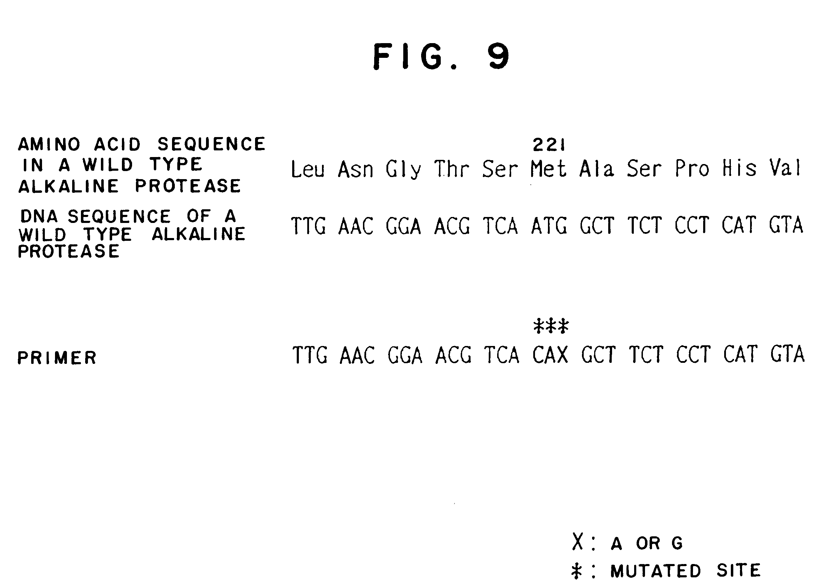

Mutation for substitution of glutamine for methionine at the 221st position of synthesized oligonucleotide (Fig. 9, 33 bases) was effected by means of in vitro mutagenesis kits (mutagen® of Bio Rad Co.).

A recombinant plasmid pULP 355 (4.5 $\mu$g, Fig. 8) where an alkaline protease gene (Fig. 1) of Bacillus licheniformis is inserted to a vector pUB 110 was digested with Kpn I and EcoR I (14 units each). Fragments obtained were subjected to agarose gel electrophoresis for fractionation to obtain a 0.97 Kb fragment (about 0.7 $\mu$g). On the other hand, RF-DNA (replicative form DNA, 2 $\mu$g) or Ml3mpl9 was digested with Kpn I and EcoR I (5 units each) and subjected to a heat-treatment at 65°C for 10 min. before precipitation was effected with ethanol in order to recover the DNAs. The both DNAs were mixed and ligated with T4 DNA ligase (4 units).

Escherichia coli JM109 was transformed with the ligated DNA (0.3 $\mu$g) and three recombinant phages which were able to form colorless plaque were obtained. Pre-cultured solution (50 $\mu$l) obtained by culturing Escherichia coli JM109 in a 2 x TY medium at 37°C overnight was inoculated in the 2 x TY medium (5 ml). To the medium was inoculated one of the three plaques formed by the recombinant phages and the medium was subjected to culturing at 37°C for 5 hours. Centrifugation was applied to twice and supernatant produced was served as a seed phage solution.

Pre-cultured solution (10 $\mu$l) obtained by culturing Escherichia coli CJ236 in a 2 x TY medium at 37°C overnight was inoculated in the 2 x TY medium (20 ml) and cultured at 37°C for 6 hours. To the culture was added the seed phage solution (100 $\mu$l) mentioned above and the phage was cultured at 37°C for 12 hours. The culture was centrifuged to produce supernatant (18 ml) containing particle of the phage. To the supernatant was added 20% by weight polyethylene glycol 6000/2.5 M NaCl (3.6 ml) and the mixture was left to stand for 30 min. in ice, until the phage was precipitated. The precipitate was recovered by centrifugation and suspended in TE buffer (200 $\mu$l, 10 mM tris-hydrochloric acid buffer solution, pH 8.0/1 mM EDTA). The suspension was left to stand in ice for 30 min. and centrifuged in order to recover supernatant to which phenol (200 $\mu$l, saturated with TE buffer solution) was added. After mixing well, a DNA was extracted. Centrifugation gave an aqueous layer to which ethanol was added in order to precipitate a single stranded DNA (about 15 $\mu$g) containing uracil.

To the single stranded phage DNA (0.1 pmol, 271 ng) were added synthetic oligonucleotide where 5'-terminal was phosphorylated (Fig. 9, 3 pmol) for mutation and 10 x annealing buffer (1 $\mu$l, 200 mM tris-hydrochloric acid buffer solution, pH 7.4/20 mM MgCl$_2$/500 mM NaCl; appendage to "mutagen"), and thereto was added sterile distilled water to make up to 10 $\mu$l. After being heated at 70°C for 5 min., the mixture was left to stand at room temperature for 40 min. until annealing was effected. While cooling with ice, 10 x synthesis buffer (1 $\mu$l, dATP/dCTP/dGTP/dTTP 5 mM each/10 mM ATP/100 mM tris-hydrochloric acid buffer, pH 7.4/50 mM MgCl$_2$/20 mM DTT: appendage to "mutagen"), T4DNA ligase (2.5 units), T4DNA polymerase (1 unit) and T4 gene 32 product (0.5 $\mu$g) were added. The mixture was left to stand in ice for 5 min. and kept at 25°C for 5 min. and at 37°C for 90 min. in order to effect an elongation reaction. TE buffer solution (90 $\mu$l) was added and the mixture was frozen at -20°C in order to cease the reaction.

Escherichia coli MV 1190 was transformed by use of the reaction mixture mentioned above (0.5 $\mu$l) by usual calcium chloride method until plaques were formed on H agar plates. Single stranded DNAs were prepared from any ten plaques. Nucleotide sequence was determined by a dideoxy sequencing method at the sites where mutation was introduced and a DNA where the desired mutation occured was selected. Thus, mutated alkaline protease gene which encodes glutamine-substituent was obtained.

Example 4

Expression of oxidation agent-resistant alkaline protease and measurement of resistance against hydrogen peroxide.

Pre-cultured solution (30 $\mu$l) obtained after Escherichia coli MV 1190 was cultured in a 2 x TY medium at 37°C overnight was inoculated in a 2 x TY medium (3 ml), and then in the medium was inoculated the selected plaque mentioned in Example 3. After culturing was made at 37°C for 5 hours, centrifugation gave a precipitate which was subjected to a usual alkaline-SDS method in order to obtain RF-DNA (20 $\mu$g). The RF-DNA (5 $\mu$g) was digested with Tth 111 I and Kpn I (50 units each) and subjected to agarose gel electrophoresis to obtain a 0.74 kb fragment (0.4 $\mu$g) containing mutated sites. On the other hand, pULP 355 (10 $\mu$g) was digested with Tth 111 I and Kpn I (50 units each) and a 5.3 kb fragment was obtained in the same manner as above.

The 5.3 fragment (0.25 $\mu$g) and the 0.74 kb fragment (0.07 $\mu$g) containing the mutated sites were mixed and ligated together by T4DNA ligase (1 unit). Bacillus subtilis PW10 was transformed in the same manner as in Example 1 with the ligated DMA.

The transformants were replicated on casein plates containing kanamycin (20 $\mu$g/ml) and cultured at 37°C for 10 - 18 hours in order to obtain halo-producing strains. One of the strains was cultured in a nutrient broth for 40 hours. Influence of hydrogen peroxide against proteases in supernatant produced on cultures was tested. That is, the supernatant after diluted to suitable concentration was left to stand at 37°C for 5 min. in the buffer solutions A and B given below, respectively:

A : 0.1 M boric acid-sodium hydroxide buffer solution, pH 9.4

B : 0.1 M boric acid-sodium hydroxide buffer solution/1 M hydrogen peroxide, pH 9.4.

To the solutions were added a substrate solution, i.e., 0.1 M tris-hydrochloric acid buffer solution, pH 8.0/5 mM CaCl$_2$ containing 0.5 mM N-succinyl-L-Ala-L-Ala-L-Pro-L-Phe-p-nitroanilide, at the final concentration, and the respective mixtures were allowed to stand at 37°C for 20 min. A reaction ceasing agent (0.35 M trichloroacetic acid/1.05 M acetic acid/0.7 M sodium acetate), was added in order to cease the reactions. Ratio of absorbance at 410 nm of the solution B to that of the solution A was calculated, the ratio being called as resistance degree against hydrogen peroxide. The resistance degree of the wild type enzyme was 14%, while that of oxidation agent-resistant enzyme OXY32Q was 94%.

Example 5

Measurement of specific activity of mutated alkaline protease

In nutrient broths (20 ml each) were inoculated Bacillus subtilis PW10 (75-L1 strain) having the surface active agent-resistant alkaline protease gene which was obtained in Example 1 and Bacillus subtilis PW10 having the oxidation agent-resistant alkaline protease gene which was obtained in Example 4, respectively. Culturings were made at 37°C for 40 hours, and then centrifugation gave supernatants. To the supernatant each was added ammonium sulfate (8.3 g), the mixture was left to stand at 4°C for one hour before centrifugation to recover precipitate. The precipitate each was dissolved in 1 ml of a buffer solution (10 mM phosphate buffer solution, pH 5.5/1 mM CaCl$_2$) and subjected to dialysis against same buffer solution. Inner solution each was recovered and flowed in a cation-exchange resin column ("Sepabeads" FP-CMI3, 0.6 ml). The column each was washed with the same buffer solution as above (3 ml each) and the alkaline protease was eluted with the same buffer solution as above containing 80 mM NaCl (2 ml each).

Protease activity and an amount of protein in the eluate each were measured as mentioned hereinunder. Specific activities of surface active agent-resistant alkaline protease (DTG 75) and oxidation agent-resistant alkaline protease (OXY32Q) were calculated.

Protease activity: On the basis of method of H. Uehara et al, J. Bacteriol., 119, 82, 1974.

Activity at 37°C under pH 10 was measured and an enzyme amount to produce tyrosine (1 $\mu$g/min) was made as one unit.

An amount of protein: On the basis of Lowry method, in terms of subtilisin.Carlsberg.

Specific activities were found 7860 units/mg protein and 7170 units/mg protein, for DTG 75 and OXY32Q, respectively, but 7680 units/mg protein for wild type enzyme.


Example 6


Preparation of protease resistant against both surface active agents and oxidation agents


A plasmid pDTG75 (5 $\mu$g) having surface active agent-resistant enzyme gene was digested with Kpn I (50 units) and Tth III I (60 units) and subjected to agarose gel electrophoresis in order to obtain a 5.3 Kb fragment (about 3.5 $\mu$g) containing a mutation site for surface active agent-resistant. On the other hand, RF-DNA (2.5 $\mu$g) of a recombinant phage having an alkaline protease gene which encodes glutamine-substituted site mentioned in Example 3 was digested with Kpn I and Tth III I (40 - 50 units each) and a 0.74 Kb fragment (about 0.2 $\mu$g) containing a mutation site for oxidation agent-resistant.

The 0.74 Kb fragment (0.07 $\mu$g) and the 5.3 Kb fragment (0.25 $\mu$g) derived from pDTG75 mentioned above were mixed and ligated with T4DNA ligase (1 unit). Bacillus subtilis PW10 was transformed with the ligated DNA, in the same manner as in Example 1. The transformant was replicated on casein plates containing kanamycin (20 $\mu$g/ml) and cultured at 37°C for 10 - 18 hours to obtain halo-producing strains. Five of the halo-producing strains were cultured in nutrient broth for 40 hours. Proteases formed in the supernatant were subjected to assay with respect to resistances against surface active agents and hydrogen peroxide, in the same manner as in Examples 1 and 4. Resistance against hydrogen peroxide of the alkaline protease (OD32Q) having both mutations for oxidation agent-resistance and surface active agent-resistance was 94% which is on the same level as OXY32Q in Example 4. Resistance against LAS of the OD32Q was 28% while 22% for the OXY32Q. Specific activity of the OD32Q measured in the same manner as in Example 5 was 6200 units/mg protein.

The present alkaline protease is suitable as auxiliary agents for detergents, since it is superior to the conventional alkaline protease with respect to resistance against surface active agents and life to keep high specific activity, so that the protease performs with high activity in detergents. The present alkaline protease is also useful as auxiliary agents for cleaners containing peroxide bleaching agents/sterilizers, or protein-removing agents used with sterilizers for contact lenses which contain hydrogen peroxide, since the protease is so resistant against oxidation agents that high activity is maintained even in the presence of oxidation agents. Furthermore, the same results as the conventional alkaline proteases are obtained even with a smaller amount of the present protease, since specific activity of the latter is on the same level as natural enzymes. In addition, the same effects as the known enzymes are obtained with the same amount of the present protease as the known enzymes, when no oxidation agents are employed. The present proteases resistant to both surface active agents and oxidation agents are useful for detergents containing both oxidation agents and surface active agents.


## Claims

1. A novel alkaline protease having the same amino acid sequence as a protease derived from Bacillus licheniformis except that the glycine and/or methionine which are the 105th and the 221st amino acids, respectively, from the N-terminal amino acid of the said protease are replaced by aspartic acid and/or glutamine, respectively.

2. Method of producing said alkaline protease defined in claim 1 by using <u>Bacillus</u> <u>subtilis</u> carrying a gene for said protease.

3. Detergents containing said alkaline protease defined in claim 1.

4. An agent for removing protein from the surface of contact lens containing said alkaline protease defined in claim 1.

**Patentansprüche**

1. Eine neue alkalische Protease, welche die gleiche Aminosäuresequenz wie eine von <u>Bacillus</u> <u>lichenifor-mis</u> abgeleitete Protease aufweist, mit der Abwandlung, daß das Glycin und/oder das Methionin, welche von der N-terminalen Aminosäure aus gezählt die 105.-, bzw. die 221. Aminosäure der besagten Protease darstellen, durch Asparaginsäure, und/oder entsprechend durch Glutamin ersetzt sind/ist.

2. Eine Methode zur Herstellung der besagten, in Anspruch 1 definierten alkalischen Protease unter Verwendung einer <u>Bacillus</u> <u>subtilis</u>-Spezies, welche ein Gen für die erwähnte Protease trägt.

3. Detergentien, welche die besagte, in Anspruch 1 definierte alkalische Protease enthalten.

4. Ein Mittel zum Entfernen von Protein(en) von der Oberfläche einer Kontaktlinse, welches die besagte, in Anspruch 1 definierte alkalische Protease enthält.

**Revendications**

1. Nouvelle protéase alcaline ayant la même séquence d'acides aminés qu'une protéase dérivée de <u>Bacillus</u> <u>licheniformis</u>, si ce n'est que la glycine et/ou la méthionine, qui sont les 105ème et les 221ème acides aminés respectivement à partir de l'acide aminé N-terminal de la protéase, sont remplacées par de l'acide aspartique et/ou de la glutamine, respectivement.

2. Procédé de préparation de la protéase alcaline définie à la revendication 1, en utilisant <u>Bacillus</u> <u>subtilis</u> portant un gène pour cette protéase.

3. Détergent contenant la protéase alcaline définie dans la revendication 1.

4. Agent d'élimination de protéine de la surface de lentilles de contact contenant la protéase alcaline définie à la revendication 1.

# FIG. IA

```
GGGACCTCTT  TCCCTGCCAG  GTTGAAGCGG  TCTATTCATA  CTTTCGAACC    50

GAATATTTTT  CTAAAACAGT  TATTAATAAC  CAATAAATTT  AAATTGGCCG   100

TTCAAAAAAA  TGGGTCTACC  ATATAATTCA  TTTTTTTTCT  ATAATAAATT   150

AACAGAATAA  TTGGAATAGA  TTATATTATC  CTTCTATTTA  AATTATTCTG   200

AATAAAGAGG  AGGAGAGTGA  GTAATGATGA  GGAAAAAGAG  TTTTTGGCTT   250

GGGATGCTGA  CGGCCTTAAT  GCTCGTGTTC  ACGATGGCAT  TCAGCGATTC   300

CGCTTCTGCT  GCTCAACCGG  CGAAAAATGT  TGAAAAGGAT  TATATTGTCG   350

GATTTAAGTC  AGGAGTGAAA  ACCGCATCTG  TCAAAAAGGA  CATCATCAAA   400

GAGAGCGGCG  GAAAAGTGGA  CAAGCAGTTT  AGAATCATCA  ACGCGGCAAA   450

AGCGAAGCTA  GACAAAGAAG  CGCTTAAGGA  AGTCAAAAAT  GATCCGGATG   500

TCGCTTATGT  GGAAGAGGAT  CATGTGGCCC  ATGCCTTGGC  GCAAACCGTT   550

CCTTACGGCA  TTCCTCTCAT  TAAAGCGGAC  AAAGTGCAGG  CTCAAGGCTT   600

TAAGGGAGCG  AATGTAAAAG  TAGCCGTCCT  GGATACAGGA  ATCCAAGCTT   650

CTCATCCGGA  CTTGAACGTA  GTCGGCGGAG  CAAGCTTTGT  GGCTGGCGAA   700
```

# FIG. IB

```
GCTTATAACA CCGACGGCAA CGGACACGGC ACGCATGTTG CCGGTACAGT   750

AGCTGCGCTT GACAATACAA CGGGTGTATT AGGCGTTGCG CCGAACGTAT   800

CCTTGTACGC GGTTAAAGTG CTGAATTCAA GCGGAAGCGG ATCTTACAGC   850

GGCATTGTAA GCGGAATCGA GTGGGCGACG ACAAACGGCA TGGATGTTAT   900

CAACATGAGC CTTGGAGGAC CATCAGGCTC AACAGCGATG AAACAGGCGG   950

TTGACAATGC ATATGCAAGA GGGGTTGTCG TTGTGGCGGC TGCTGGGAAC   1000

AGCGGATCTT CAGGAAACAC GAATACAATC GGCTATCCTG CGAAATACGA   1050

CTCTGTCATC GCAGTTGGCG CGGTAGACTC TAACAGCAAC AGAGCTTCAT   1100

TTTCCAGCGT CGGAGCAGAG CTTGAAGTCA TGGCTCCTGG CGCAGGCGTG   1150

TACAGCACTT ACCCAACCAG CACTTATGCA ACATTGAACG GAACGTCAAT   1200

GGCTTCTCCT CATGTAGCGG GAGCAGCAGC TTTGATCTTG TCAAAACATC   1250

CGAACCTTTC AGCTTCACAA GTCCGCAACC GTCTCTCCAG TACGGCGACT   1300

TATTTGGGAA GCTCCTTCTA CTATGGAAAA GGTCTGATCA ATGTCGAAGC   1350

TGCCGCTCAA TAACATATTC TAACAAATGG CATATAGAAA AAAGCTAGTG   1400
```

14

# FIG. IC

TTTTTAGCAC TAGCTTTTTC TTCATTCAGT TGAAGACTGT TCAATATTTT 1450

GAATCCGTTC CATTATGGTC GGATGGCCGT ATTTAAAAAT CTTGACGAGA 1500

AACGGCGGGT TCGCCTCGCT CAACCCGGCT TTTGAGAGCT CTTGAAAAGT 1550

CGGCAACCGC TGCATCGTGT GTTCGTCAGT CAATCGCATA CTGGTCAGCG 1600

GCTTTTTCCT GATGCCTCGA AACTGCGTTT GTAAATGGAG AAGACGCGAA 1650

AGAGAGTGAC CCCCATCAGC ATCAGGAGAA GCGGGAGTGC GGCAAGATCG 1700

GATTTTCCTG CAATATGAAG GCTTTTTCCA TAGCGGCCGA TGATCCGCTT 1750

GTACAGCTTG TCGATCACAT AAAAGACGGC AAGGGATAAA AGCAGGTACC 1800

CGCCGAGTCC TATGTAAACA TGCTTCATCA CATAGTGCCC CATTTCGTGC 1850

GCCATAATGA ATAAAATTTC CGGCTCATCC AGCTTGTTCA GCGTGGTATC 1900

CCAGAGCACA ATCCTTTTAT TGGCGCCGAT CCCTGTCACA TAAGCATTTA 1950

ACGCATTCGT TTTTTCAGAC ATGTTCACTT CATAGACGTG ATTCGCAGGA 2000

ATATCTGCCT GATCGGCCAG CTTCAGAATG CTTTGTTCGA GATC      2044

15

# FIG. 2A

Ala Gln Thr Val Pro Tyr Gly Ile Pro Leu Ile Lys Ala Asp Lys Val   16
                 5                10              15

Gln Ala Gln Gly Phe Lys Gly Ala Asn Val Lys Val Ala Val Leu Asp   32
           20             25              30

Thr Gly Ile Gln Ala Ser His Pro Asp Leu Asn Val Val Gly Gly Ala   48
        35             40            45

Ser Phe Val Ala Gly Glu Ala Tyr Asn Thr Asp Gly Asn Gly His Gly   64
       50           55            60

Thr His Val Ala Gly Thr Val Ala Ala Leu Asp Asn Thr Thr Gly Val   80
65             70           75           80

Leu Gly Val Ala Pro Asn Val Ser Leu Tyr Ala Val Lys Val Leu Asn   96
        85             90           95

Ser Ser Gly Ser Gly Ser Tyr Ser Gly Ile Val Ser Gly Ile Glu Trp   112
        100          105        110

Ala Thr Thr Asn Gly Met Asp Val Ile Asn Met Ser Leu Gly Gly Pro   128
        115          120        125

Ser Gly Ser Thr Ala Met Lys Gln Ala Val Asp Asn Ala Tyr Ala Arg   144
       130          135         140

EP 0 416 967 B1

# FIG. 2B

Gly Val Val Val Val Ala Ala Ala Gly Asn Ser Gly Ser Ser Gly Asn   160

Thr Asn Thr Ile Gly Tyr Pro Ala Lys Tyr Asp Ser Val Ile Ala Val   176

Gly Ala Val Asp Ser Asn Ser Asn Arg Ala Ser Phe Ser Ser Val Gly   192

Ala Glu Leu Glu Val Met Ala Pro Gly Ala Gly Val Tyr Ser Thr Tyr   208

Pro Thr Ser Thr Tyr Ala Thr Leu Asn Gly Thr Ser Met Ala Ser Pro   224

His Val Ala Gly Ala Ala Ala Leu Ile Leu Ser Lys His Pro Asn Leu   240

Ser Ala Ser Gln Val Arg Asn Arg Leu Ser Ser Thr Ala Thr Tyr Leu   256

Gly Ser Ser Phe Tyr Tyr Gly Lys Gly Leu Ile Asn Val Glu Ala Ala   272

Ala Gln                                                            274

EP 0 416 967 B1

# FIG. 3A

Ala Gln Thr Val Pro Tyr Gly Ile Pro Leu Ile Lys Ala Asp Lys Val  16
            5                10             15

Gln Ala Gln Gly Phe Lys Gly Ala Asn Val Lys Val Ala Val Leu Asp  32
          20             25             30

Thr Gly Ile Gln Ala Ser His Pro Asp Leu Asn Val Val Gly Gly Ala  48
        35             40             45

Ser Phe Val Ala Gly Glu Ala Tyr Asn Thr Asp Gly Asn Gly His Gly  64
     50            55             60

Thr His Val Ala Gly Thr Val Ala Ala Leu Asp Asn Thr Thr Gly Val  80
65              70           75             80

Leu Gly Val Ala Pro Asn Val Ser Leu Tyr Ala Val Lys Val Leu Asn  96
        85             90             95

Ser Ser Gly Ser Gly Ser Tyr Ser Asp Ile Val Ser Gly Ile Glu Trp  112
        100           105           110

Ala Thr Thr Asn Gly Met Asp Val Ile Asn Met Ser Leu Gly Gly Pro  128
      115            120          125

Ser Gly Ser Thr Ala Met Lys Gln Ala Val Asp Asn Ala Tyr Ala Arg  144
   130            135            140

EP 0 416 967 B1

## FIG. 3B

Gly Val Val Val Val Ala Ala Ala Gly Asn Ser Gly Ser Ser Gly Asn  160

Thr Asn Thr Ile Gly Tyr Pro Ala Lys Tyr Asp Ser Val Ile Ala Val  176

Gly Ala Val Asp Ser Asn Ser Asn Arg Ala Ser Phe Ser Ser Val Gly  192

Ala Glu Leu Glu Val Met Ala Pro Gly Ala Gly Val Tyr Ser Thr Tyr  208

Pro Thr Ser Thr Tyr Ala Thr Leu Asn Gly Thr Ser Met Ala Ser Pro  224

His Val Ala Gly Ala Ala Ala Leu Ile Leu Ser Lys His Pro Asn Leu  240

Ser Ala Ser Gln Val Arg Asn Arg Leu Ser Ser Thr Ala Thr Tyr Leu  256

Gly Ser Ser Phe Tyr Tyr Gly Lys Gly Leu Ile Asn Val Glu Ala Ala  272

Ala Gln  274

EP 0 416 967 B1

# FIG. 4A

Ala Gln Thr Val Pro Tyr Gly Ile Pro Leu Ile Lys Ala Asp Lys Val 16
　　　　　　　5　　　　　　　　　　10　　　　　　　　15

Gln Ala Gln Gly Phe Lys Gly Ala Asn Val Lys Val Ala Val Leu Asp 32
　　　　　20　　　　　　　　25　　　　　　　　30

Thr Gly Ile Gln Ala Ser His Pro Asp Leu Asn Val Val Gly Gly Ala 48
　　　　　35　　　　　　　　40　　　　　　　　45

Ser Phe Val Ala Gly Glu Ala Tyr Asn Thr Asp Gly Asn Gly His Gly 64
　　　50　　　　　　　　55　　　　　　　　60

Thr His Val Ala Gly Thr Val Ala Ala Leu Asp Asn Thr Thr Gly Val 80
65　　　　　　　　70　　　　　　　　75　　　　　　　　80

Leu Gly Val Ala Pro Asn Val Ser Leu Tyr Ala Val Lys Val Leu Asn 96
　　　　　　85　　　　　　　　90　　　　　　　　95

Ser Ser Gly Ser Gly Ser Tyr Ser Gly Ile Val Ser Gly Ile Glu Trp 112
　　　　　100　　　　　　　　105　　　　　　　110

Ala Thr Thr Asn Gly Met Asp Val Ile Asn Met Ser Leu Gly Gly Pro 128
　　　　　115　　　　　　　.120　　　　　　　125

Ser Gly Ser Thr Ala Met Lys Gln Ala Val Asp Asn Ala Tyr Ala Arg 144
　　　130　　　　　　　135　　　　　　　140

# FIG. 4B

Gly Val Val Val Val Ala Ala Ala Gly Asn Ser Gly Ser Ser Gly Asn   160
145             150         155               160

Thr Asn Thr Ile Gly Tyr Pro Ala Lys Tyr Asp Ser Val Ile Ala Val   176
165           170           175

Gly Ala Val Asp Ser Asn Ser Asn Arg Ala Ser Phe Ser Ser Val Gly   192
180         185          190

Ala Glu Leu Glu Val Met Ala Pro Gly Ala Gly Val Tyr Ser Thr Tyr   208
195          200        205

Pro Thr Ser Thr Tyr Ala Thr Leu Asn Gly Thr Ser Gln Ala Ser Pro   224
210          215        220

His Val Ala Gly Ala Ala Ala Leu Ile Leu Ser Lys His Pro Asn Leu   240
225         230       235         240

Ser Ala Ser Gln Val Arg Asn Arg Leu Ser Ser Thr Ala Thr Tyr Leu   256
245         250         255

Gly Ser Ser Phe Tyr Tyr Gly Lys Gly Leu Ile Asn Val Glu Ala Ala   272
260        265         270

Ala Gln                                   274
274

EP 0 416 967 B1

# FIG. 5A

Ala Gln Thr Val Pro Tyr Gly Ile Pro Leu Ile Lys Ala Asp Lys Val 16
                   5              10            15

Gln Ala Gln Gly Phe Lys Gly Ala Asn Val Lys Val Ala Val Leu Asp 32
           20            25            30

Thr Gly Ile Gln Ala Ser His Pro Asp Leu Asn Val Val Gly Gly Ala 48
         35           40          45

Ser Phe Val Ala Gly Glu Ala Tyr Asn Thr Asp Gly Asn Gly His Gly 64
    50             55           60

Thr His Val Ala Gly Thr Val Ala Ala Leu Asp Asn Thr Thr Gly Val 80
65              70          75            80

Leu Gly Val Ala Pro Asn Val Ser Leu Tyr Ala Val Lys Val Leu Asn 96
         85           90          95

Ser Ser Gly Ser Gly Ser Tyr Ser Asp Ile Val Ser Gly Ile Glu Trp 112
         100         105         110

Ala Thr Thr Asn Gly Met Asp Val Ile Asn Met Ser Leu Gly Gly Pro 128
        115          120         125

Ser Gly Ser Thr Ala Met Lys Gln Ala Val Asp Asn Ala Tyr Ala Arg 144
    130            135          140

EP 0 416 967 B1

# FIG. 5B

Gly Val Val Val Val Ala Ala Ala Gly Asn Ser Gly Ser Ser Gly Asn 160
145 150 155 160

Thr Asn Thr Ile Gly Tyr Pro Ala Lys Tyr Asp Ser Val Ile Ala Val 176
165 170 175

Gly Ala Val Asp Ser Asn Ser Asn Arg Ala Ser Phe Ser Ser Val Gly 192
180 185 190

Ala Glu Leu Glu Val Met Ala Pro Gly Ala Gly Val Tyr Ser Thr Tyr 208
195 200 205

Pro Thr Ser Thr Tyr Ala Thr Leu Asn Gly Thr Ser Gln Ala Ser Pro 224
210 215 220

His Val Ala Gly Ala Ala Ala Leu Ile Leu Ser Lys His Pro Asn Leu 240
225 230 235 240

Ser Ala Ser Gln Val Arg Asn Arg Leu Ser Ser Thr Ala Thr Tyr Leu 256
245 250 255

Gly Ser Ser Phe Tyr Tyr Gly Lys Gly Leu Ile Asn Val Glu Ala Ala 272
260 265 270

Ala Gln 274
274

EP 0 416 967 B1

FIG. 6

pHLP352 6.9 kb

EcoRI  SmaI  EcoRI  BgIII  Tcr  Ampr

FIG. 7

M13LP1 9.2 kb

HindIII  XbaI (BamHI/BgIII)  EcoRI  SmaI  EcoRI

FIG. 8

pULP355 6.0kb

XbaI  EcoRI  (BamHI/BgIII)  KpnI  TthIIII  EcoRI  (PvuII/SmaI)  Kmr  BgIII

# FIG. 9

AMINO ACID SEQUENCE
IN A WILD TYPE
ALKALINE PROTEASE

221
Leu Asn Gly Thr Ser Met Ala Ser Pro His Val

DNA SEQUENCE OF A
WILD TYPE ALKALINE
PROTEASE

TTG AAC GGA ACG TCA ATG GCT TCT CCT CAT GTA

\*\*\*

PRIMER

TTG AAC GGA ACG TCA CAX GCT TCT CCT CAT GTA

X : A OR G

\* : MUTATED SITE

EP 0 416 967 B1